# FASCICULE DE BREVET EUROPEEN

(11) **EP 2 337 581 B1**
(45) Date de publication et mention de la délivrance du brevet: **23.08.2017**
(21) Numéro de dépôt: 09741397.5
(22) Date de dépôt: 10.09.2009
(51) Int. Cl.: A61K 39/21, C12N 15/62, C12N 15/863

(54) **CONSTRUCTION D'UN GÈNE SYNTHÉTIQUE CODANT POUR GAG DE VIH1 ET SON UTILISATION POUR L'OBTENTION DE VACCINS ANTI-VIH-1**
SYNTHETISCHES GEN, DAS FÜR DAS GAG-PROTEIN VON HIV-1 KODIERT UND DESSEN VERWENDUNG ZUR PRODUKTION VON IMPFSTOFFE GEGEN HIV-1
SYNTHETIC GENE CODING FOR THE GAG PROTEIN OF HIV-1 AND USE THEREOF FOR OBTAINING ANTI-HIV-1 VACCINES

(30) Priorité: 10.09.2008 EP 08290849
(43) Date de publication de la demande: 29.06.2011
(73) Titulaire: Institut National de la Santé et de la Recherche Médicale, 75013 Paris (FR); Centre National de la Recherche Scientifique, 75016 Paris (FR); Université Paris-Est Créteil Val de Marne, 94000 Créteil (FR)
(72) Inventeur: GUILLET, Jean-Gérard, décédé (FR); LEVY, Yves, F-94010 Creteil (FR); BALLOUL, Jean Marc, F-67405 Illkirch Graffenstaden Cedex (FR); SCHMITT, Doris, F-67405 Illkirch Graffenstaden Cedex (FR)
(74) Mandataire: Lavoix
(86) Numéro de dépôt international: PCT/FR2009/051704
(87) Numéro de publication internationale: WO 2010/029260

(56) Documents cités:
- EP-A- 1 921 146
- WO-A-2005/030964
- PR. YVES LEVY & ANNE DE SAUNIÈRE: "La recherche vaccinale de l'ANRS - Prospective 2007-2010"[Online] 9 février 2007 (2007-02-09), XP002511934 Extrait de l'Internet: URL:http://www.anrs.fr/index.php/anrs/cont ent/download/1685/10599/file/6-La%20recher che%20vaccinale%20de%20l'ANRS.pps> [extrait le 2009-01-23]
- SMITH JAMES M ET AL: "DNA/MVA vaccine for HIV type 1: Effects of codon-optimization and the expression of aggregates or virus-like particles on the immunogenicity of the DNA prime" AIDS RESEARCH AND HUMAN RETROVIRUSES, vol. 20, no. 12, décembre 2004 (2004-12), pages 1335-1347, XP002511935 ISSN: 0889-2229

## Description

La présente demande décrit un gène synthétique codant pour la protéine Gag (p55) de VIH-1 (virus de l'immunodéficience humaine), et son utilisation pour l'obtention de vaccins anti-VIH.

Les vaccins anti-VIH actuellement en cours de développement sont basés sur différentes approches (pour revue cf. par exemple (NABEL, Nature, 410, 1002-7, 2001) parmi lesquelles on citera l'utilisation de vecteurs d'expression, notamment des vecteurs viraux recombinants, dans lesquels sont insérés des portions du génome de VIH codant pour des polypeptides portant des épitopes reconnus par des anticorps, ou par des lymphocytes T cytotoxiques (CTLs), anti-VIH, et donc potentiellement inducteurs d'une réponse immunitaire humorale et/ou cellulaire anti-VIH.

Parmi les régions du génome de VIH les plus utilisées dans ce cadre, figurent celles dérivées du gène *gag.* La protéine Gag constitue notamment un immunogène particulièrement intéressant pour l'induction, non seulement d'une réponse anticorps, mais également d'une réponse CTL.

Généralement, afin d'induire la réponse immunitaire la plus large possible, on associe dans un même vecteur différentes portions du génome de VIH, dont l'expression à partir de ce vecteur s'effectue sous forme d'une protéine de fusion. On citera à titre d'exemples, des protéines de fusion associant la protéine Gag avec des régions de la protéine Pol, de la protéine Nef, et/ou la protéine Env.

Un problème rencontré lors de l'utilisation de ces vecteurs d'expression réside dans le faible niveau d'expression des gènes de VIH qui y sont insérés, et dans le manque de stabilité de cette expression.

Une approche actuellement utilisée pour augmenter le niveau d'expression des gènes de VIH consiste à optimiser leur séquence codante, pour supprimer les séquences nucléotidiques inhibitrices (INS) riches en AT, présentes notamment dans les gènes *gag, pol* et *env* et pour modifier l'usage des codons, en remplaçant certains des codons initiaux de VIH par des codons synonymes préférentiellement utilisés dans les cellules humaines (QIU et al., J Virol, 73, 9145-52, 1999; Zur Megede et al., J Virol, 74, 2628-35, 2000; Kotsopoulou et al., J Virol, 74, 4839-52, 2000).

Cette approche permet d'augmenter le niveau d'expression, mais la stabilité de cette expression demeure encore problématique, notamment dans le cas du gène *gag.*

Les Inventeurs ont émis l'hypothèse que la présence de nombreux motifs poly C, poly G ou poly GC dans les séquences utilisées pour exprimer la protéine *gag* jouait un rôle dans ce manque de stabilité, et ont procédé à une optimisation supplémentaire en vue de supprimer les motifs poly C ou poly G de taille supérieures à 3 nucléotides et les motifs poly GC de taille supérieure à 8 nucléotides.

La présente invention est définie par les revendications.

La présente demande décrit en conséquence un polynucléotide codant pour la protéine Gag (p55) de VIH-1, caractérisé en ce qu'il est défini par la séquence SEQ ID NO: 1.

La présente demande décrit également un polynucléotide recombinant, comprenant la séquence SEQ ID NO:1. Avantageusement, ledit polynucléotide recombinant code pour une protéine de fusion comprenant la protéine Gag (p55) de VIH-1, fusionnée avec un ou plusieurs autre(s) polypeptide (s) de VIH.

De préférence, le(s)dit(s) autre(s) polypeptide (s) de VIH est (sont) choisi(s) parmi la protéine Pol, la protéine Nef, la protéine Env, ou tout fragment desdites protéines portant au moins un épitope reconnu par des anticorps anti-VIH, ou par des lymphocytes T cytotoxiques CTLs anti-VIH. A titre d'exemples non-limitatifs de tels fragments, on citera des fragments de la protéine Pol homologues aux fragments 172-219, 325-383, et 461-519 de la protéine Pol de l'isolat VIH-1 Bru/LAI, et les fragments de la protéine Nef homologues aux fragments 66-147 et 182-206 de la protéine Nef de l'isolat VIH-1 Bru/LAI. L'isolat VIH-1 Bru/LAI est répertorié dans le catalogue de Los Alamos sous le numéro d'accession K02013. Sauf précision contraire, la numérotation des acides aminés des séquences des protéines de cet isolat est utilisée ici comme référence, lorsque des fragments peptidiques sont définis par leur localisation par rapport à la séquence d'une protéine de VIH.

Selon un mode de réalisation préféré décrit dans la présente demande, ledit polynucléotide recombinant est défini par la séquence SEQ ID NO: 2.

Il code pour la protéine de fusion gag-nef-pol, définie par la séquence SEQ ID NO: 3.

Cette protéine de fusion, qui est également décrite dans la présente demande, comprend, outre la totalité de la protéine Gag (p55) (acides aminés 1-512 de la séquence SEQ ID NO: 3), des fragments correspondant aux fragments 461-519, 325-383, et 172-219 de la protéine Pol de l'isolat VIH-1 Bru/LAI (ces fragments sont représentés respectivement par les acides aminés 517-575, 605-663, et 748-795 de la séquence SEQ ID NO: 3) et des fragments correspondant aux fragments 182-206 et 66-147 de la protéine Nef de l'isolat VIH-1 Bru/LAI (ces fragments sont représentés respectivement par les acides aminés 577-601 et 665-746 de la séquence SEQ ID NO: 3).

La présente demande décrit également un vecteur recombinant contenant un polynucléotide décrit dans la demande.

De préférence, un vecteur recombinant tel que décrit dans la demande est un vecteur d'expression ; avantageusement il s'agit d'un vecteur vaccinal.

De très nombreux vecteurs vaccinaux, utilisables notamment dans le cadre de la vaccination anti-VIH, sont connus en eux-mêmes. A titres d'exemples, on citera les vecteurs à ADN nu, ainsi que les vecteurs viraux recombinants. Parmi ces derniers, on mentionnera notamment : les vecteurs dérivés de poxvirus, par exemple du virus de la vaccine, tels que le NYVAC (New-York vaccine), et le MVA (virus Ankara modifié), ou de poxvirus aviaires tels que le canarypox ; les vecteurs dérivés d'adénovirus, tels que l'adénovirus de type 5 (Ad5) ; des vecteurs dérivés d'alphavirus, de myxomavirus, ou de virus herpès défectifs.

Selon un mode de réalisation préféré décrit dans la présente demande, ledit vecteur est dérivé d'un virus de la vaccine, et avantageusement, du virus MVA. Ce virus, qui dérive de la souche Ankara du virus de la vaccine, a été fortement atténué par 574 passages sur des fibroblastes embryonnaires de poulet, à la suite desquels il a perdu la capacité de se répliquer efficacement dans la plupart des cellules de mammifères (on n'observe une réplication efficace que dans les fibroblastes embryonnaires de poulet et les cellules BHK-21). Cette atténuation résulte de plusieurs excisions (excisions I, II, III, IV, et VI) dans le génome viral (MEYER et al., J Gen Virol, 72 (Pt 5), 1031-8, 1991). On peut insérer du matériel génétique exogène au niveau de l'une quelconque de ces excisions.

La présente invention a également pour objet l'utilisation d'un vecteur recombinant conforme à l'invention, pour l'obtention d'un vaccin anti-VIH.

L'invention concerne également une composition immunogène ou vaccinale comprenant un vecteur recombinant selon l'invention, et un véhicule pharmaceutiquement acceptable. Des véhicules pharmaceutiquement acceptables sont bien connus de l'homme du métier. Il peut être fait référence notamment à l'ouvrage Remington: The Science and Practice of Pharmacy, 21e édition, Lippincott Williams & Wilkins.Dans ce cadre, ledit vecteur peut avantageusement être utilisé en association avec les lipopeptides, par exemple des lipopeptides palmitoylés. Les lipopeptides sont constitués de fragments immunogènes de protéines du VIH liés de façon covalente à une chaîne lipidique. Des lipopeptides utilisables dans le cadre de la présente invention sont par exemple décrits dans les Demandes EP0491628, ou WO 99/51630. Avantageusement, les fragments immunogènes de protéines du VIH utilisés sont des fragments homologues aux fragments 17-35 et 253-284 de la protéine Gag, au fragment 325-355 de la protéine Pol et aux fragments 66-97 et 116-145 de la protéine Nef.

Avantageusement, les vecteurs recombinants conformes à l'invention peuvent être utilisés en association avec des lipopeptides dans le cadre d'une vaccination de type « prime-boost », (comprenant une primo-immunisation avec un vecteur conforme à l'invention, suivie d'un rappel avec lesdits lipopeptides, ou inversement, une primo-immunisation avec les lipopeptides, suivie d'un rappel avec un vecteur conforme à l'invention).

Selon un autre aspect, la demande décrit une méthode pour induire une réponse immune chez un sujet qui le nécessite, ladite méthode comprenant l'administration d'un vecteur recombinant décrit dans la demande.

La demande décrit également une méthode de prévention ou traitement d'une infection par le VIH comprenant l'administration à un sujet qui le nécessite d'un vecteur recombinant décrit dans la demande. En particulier l'infection peut être une infection par le VIH-1. Le vecteur recombinant est préférentiellement administré en une quantité immunologiquement efficace, c'est-à-dire une quantité suffisante pour induire une réponse immunologique protectrice ou thérapeutique chez le sujet ayant reçu le vecteur, ou la composition immunogène ou vaccinale le comprenant.

La présente invention est illustrée, de manière non limitative, par les figures et les exemples qui suivent.

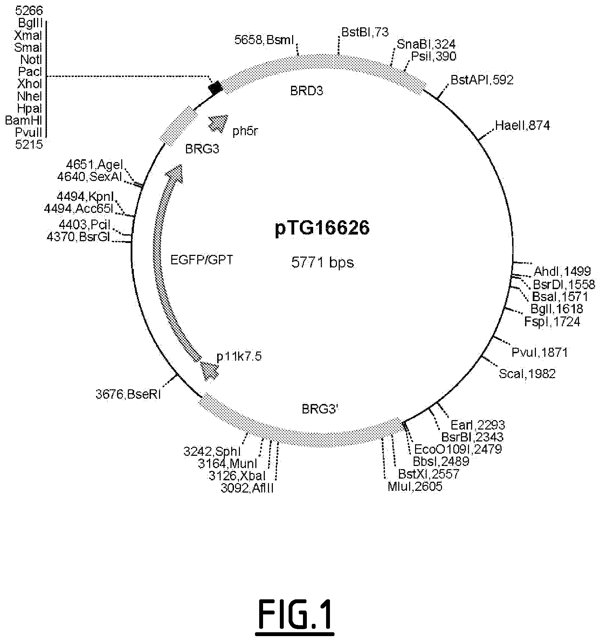

La Figure 1 schématise le vecteur de transfert pTG16626. BRD3 : Bras de recombinaison droit (séquence située en aval de l'excision III du MVA) ; BRG3 : Bras de recombinaison gauche (séquence située en amont de la délétion III du MVA) ; GPT/EGFP : marqueur de sélection ; BRG3' : séquence de répétition de BRG3.

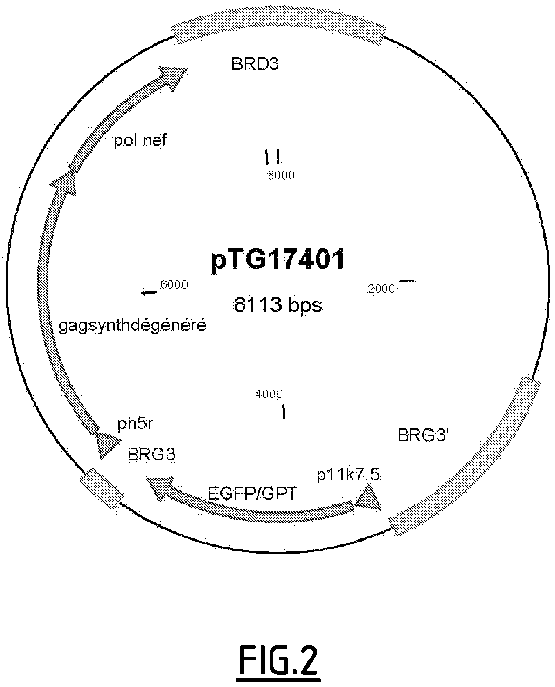

La Figure 2 schématise le vecteur pTG17401, résultant de l'insertion de la construction *gag(dégénéré)-pol-nef* dans pTG16626.

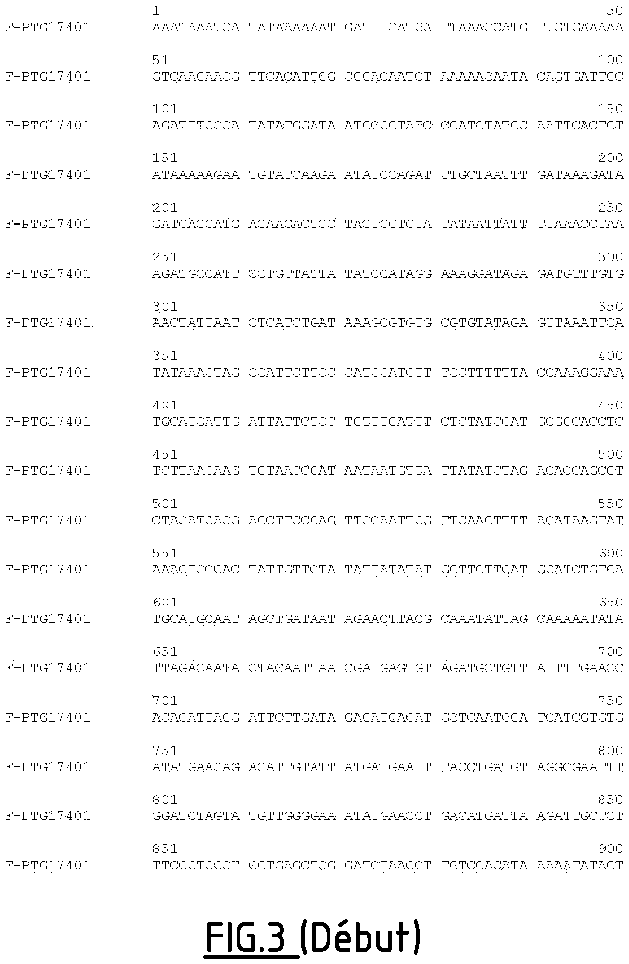

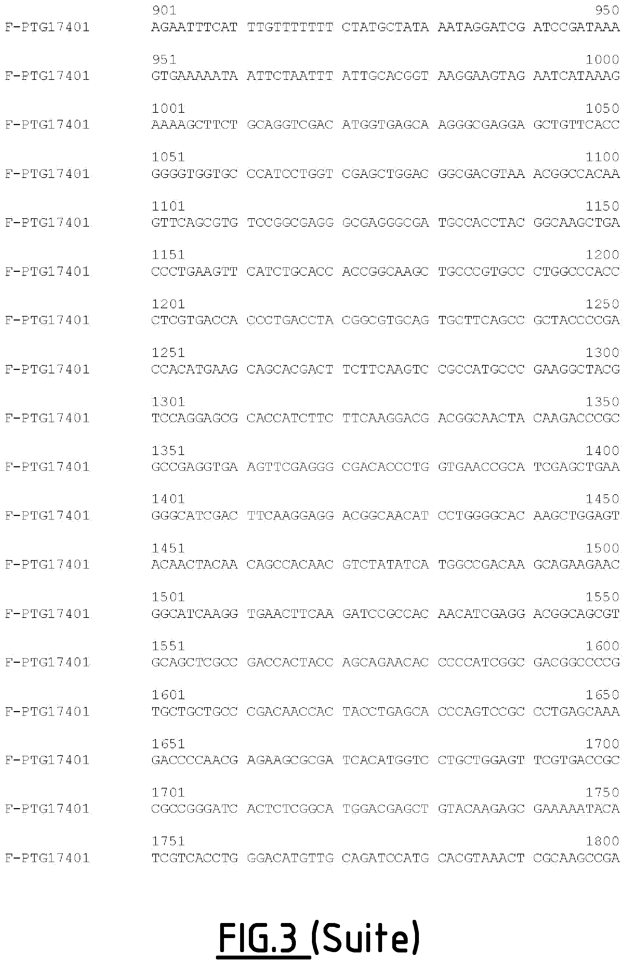

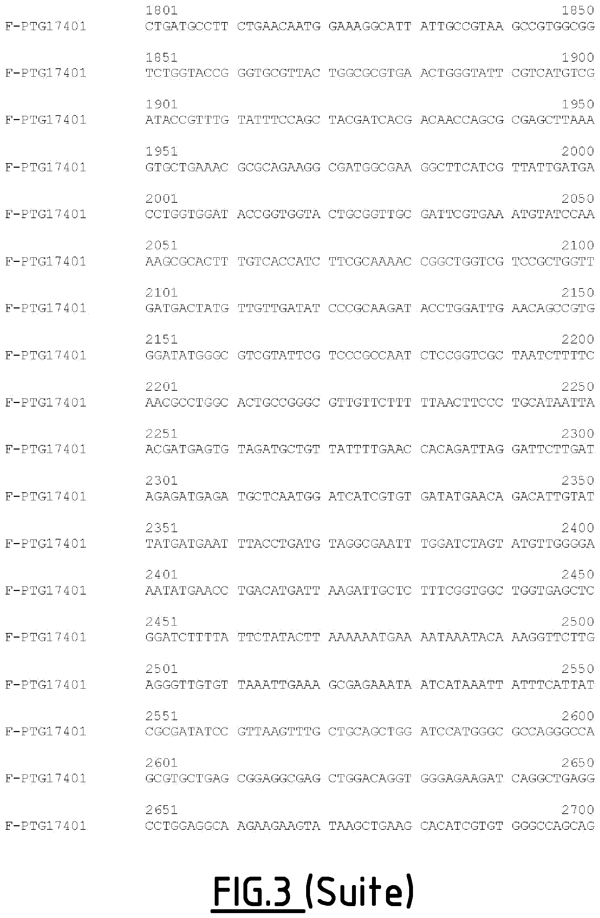

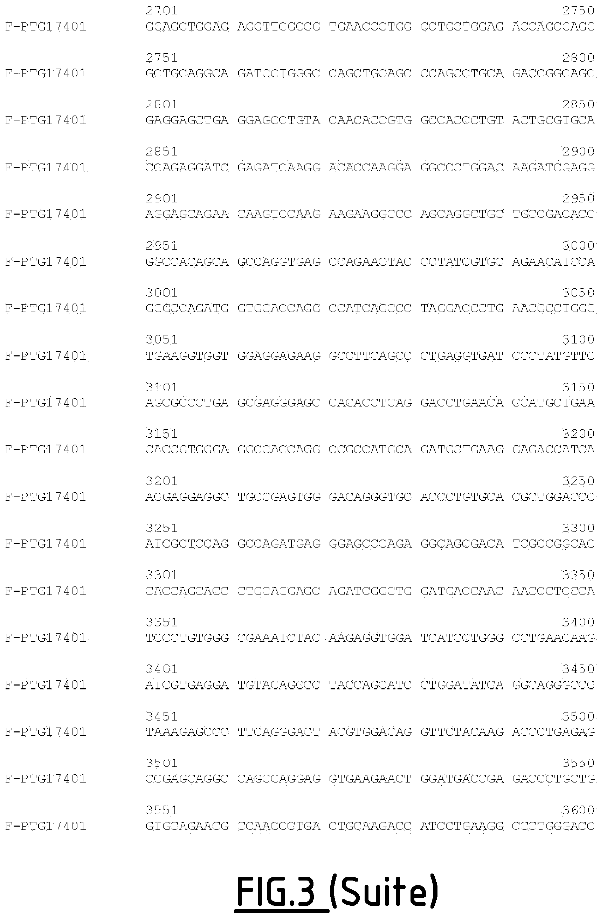

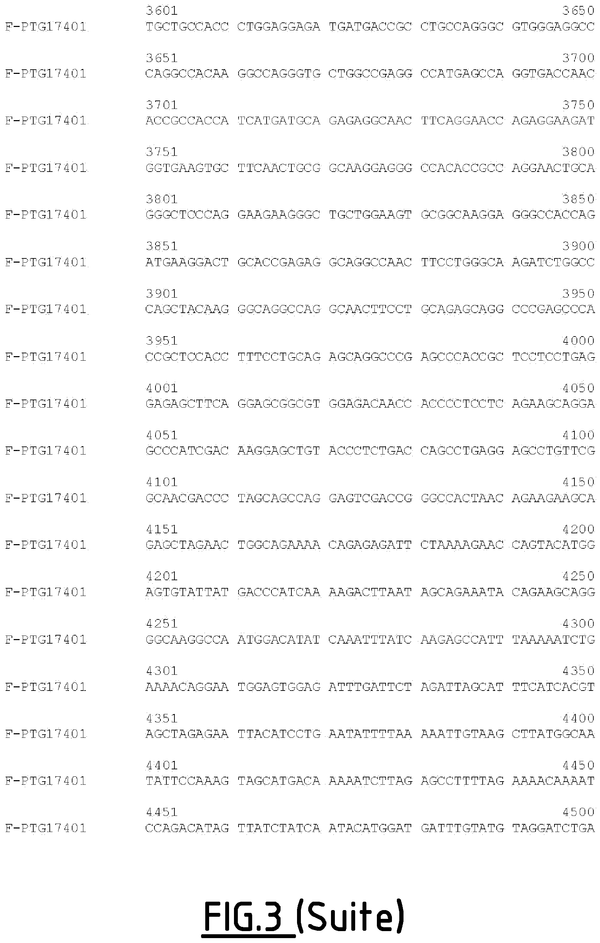

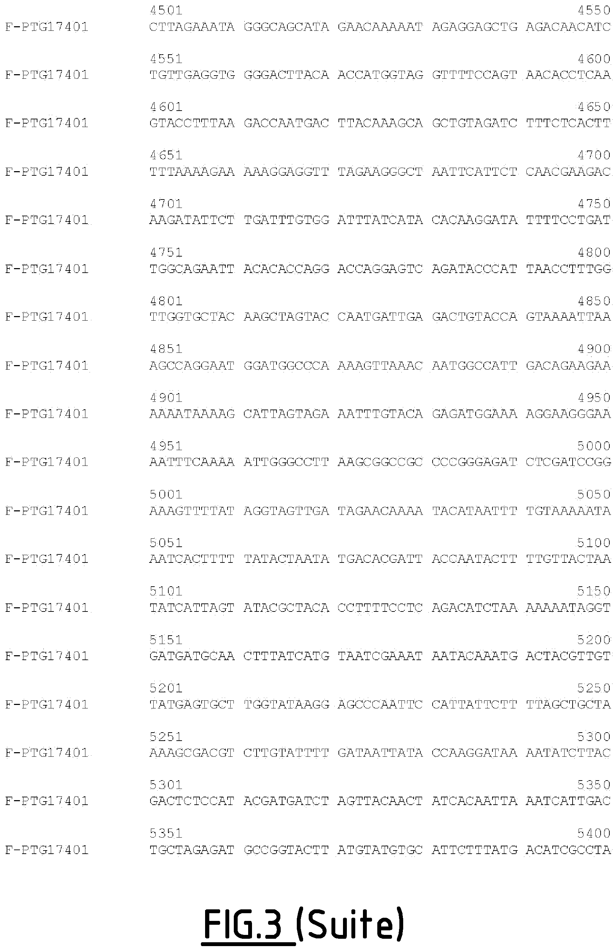

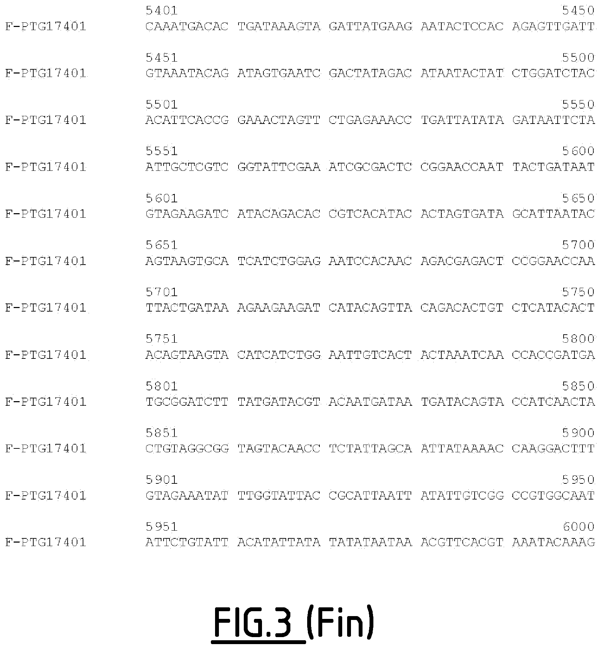

La Figure 3 représente la séquence de la région du vecteur pTG17401 comprenant la séquence BRG3', la cassette de sélection contenant le gène de fusion EGFP/GPT sous contrôle du promoteur p11K7.5K, la séquence BRG3, le gène de fusion *gag(dégénéré)-pol-nef* sous contrôle du promoteur ph5R, et la séquence BRD3.

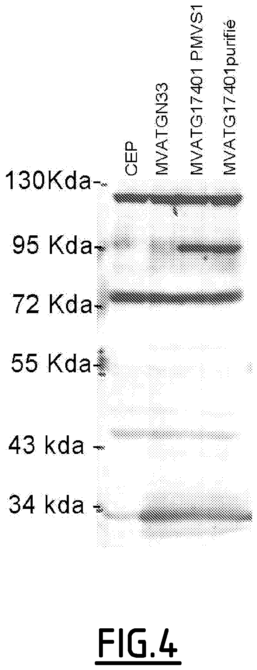

La Figure 4 représente les résultats de l'analyse par Western Blot de l'expression de *gag(dégénéré)polnef* dans des cellules embryonnaires de poulet : CEP : témoin (cellules non infectées) ; MVATGN33 : cellules infectées par MVATGN33 ; MVATG17401 PMVS1 : cellules infectées avec le stock primaire PMVS1 de MVATG17401 ; MVATG17401 purifié : cellules infectées avec le virus MVATG17401 purifié.

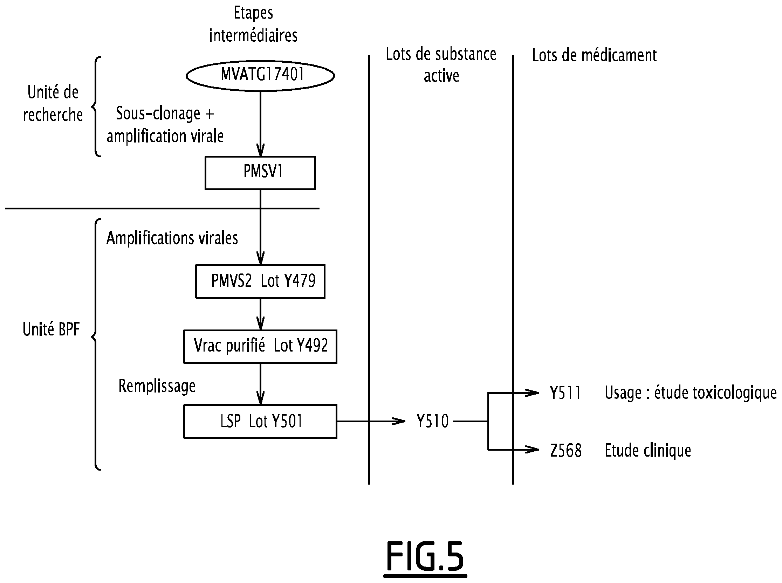

La Figure 5 représente la filiation entre les différents lots de virus après sous-clonage et amplification du virus MVATG17401.

### EXEMPLE 1 : OBTENTION D'UNE SEQUENCE GAG DEGENEREE, ET D'UNE CONSTRUCTION GAG(DEGENERE)-POL-NEF CONTENANT CETTE SEQUENCE.

La séquence dégénérée de *gag* (SEQ ID NO: 1) a été fractionnée en deux parties, qui ont été assemblées individuellement par PCR à partir d'oligonucléotides synthétiques. Cet assemblage a généré deux fragments, *Bam*HI*-Eco*RV (fragment 1) et *Eco*RV*-Sal*I (fragment 2), les sites *Eco*RV et *Sal*I faisant partie intégrante de la séquence de *gag* modifiée.

Les deux fragments *Bam*HI*-Eco*RV et *Eco*RV*-Sal*I ainsi obtenus, ainsi qu'un fragment *Sal*I*-Not*I de 851 pb, contenant des séquences codant pour des fragments de protéines Pol et Nef sont liés entre eux par la ligase T4, et le fragment *gag(dégénéré)-pol-nef* résultant est cloné entre les sites *Bam*HI et *Not*I du vecteur de transfert pTG16626, décrit ci-après, et schématisé sur la Figure 1.

Le vecteur de transfert pTG16626 contient une cassette d'expression constituée par un lieur multisite permettant l'insertion d'une séquence exogène sous contrôle transcriptionnel du promoteur ph5R (Promoteur tardif/précoce du virus de la vaccine, SMITH et al., Vaccine.; 11, 43-53, 1993). Cette cassette est encadrée par les bras de recombinaison BRD3 et BRG3, qui correspondent aux séquences flanquant la zone d'excision III du virus MVA, et permettront l'insertion de la cassette dans le génome du MVA au niveau de cette zone d'excision. Ce vecteur contient en outre une cassette de sélection, basée sur l'expression du gène de fusion EGFP/GPT (enhanced green fluorescent protein/xanthine-guanine phosphoribosyl transférase de E. Coli) placé sous contrôle du promoteur vaccinal p11K75 (promoteur précoce-tardif synthétique, résultant de la fusion du promoteur tardif p11K et du promoteur précoce p7.5K , décrit par exemple dans la Demande EP1146125). La synthèse de xanthine-guanine phosphoribosyl transférase permet la formation de plages virales de MVA recombinant en milieu sélectif contenant de la xanthine, de l'acide mycophénolique et de l'hypoxanthine (FALKNER and MOSS, J Virol, 62, 1849-54, 1988) et la synthèse de l'EGFP permet la visualisation de plages fluorescentes. Cette cassette de sélection est encadrée d'une part par la séquence BRG3, et d'autre part par une séquence homologue BRG3'. La recombinaison intragénique entre ces deux séquences homologues permet l'élimination de la cassette de sélection après plusieurs passages du MVA recombinant en milieu non-sélectif.

L'insertion du fragment *gag(dégénéré)pol-nef* dans le vecteur pTG16626 ouvert par *Bam*HI *Not*I génère le vecteur pTG17401, qui est schématisé sur la Figure 2.

La séquence de la région du vecteur pTG17401, comprenant la séquence BRG3', la cassette de sélection contenant le gène de fusion EGFP/GPT sous contrôle du promoteur p11K7.5K, la séquence BRG3, le gène de fusion *gag(dégénéré)-pol-nef* sous contrôle du promoteur ph5R, et la séquence BRD3, est représentée sur la Figure 3 (SEQ ID NO: 4).

### EXEMPLE 2 : GENERATION D'UN VIRUS DE LA VACCINE RECOMBINANT EXPRIMANT LE GENE DE FUSION GAG(DEGENERE)-POL-NEF

La génération de virus de la vaccine recombinant se fait par recombinaison homologue entre le plasmide de transfert pTG17401 et un virus MVA (MVATGN33). Les virus recombinants sont sélectionnés pour leur capacité à former des plages de lyse en présence d'acide mycophénolique.

Les cellules embryonnaires de poulet utilisées pour la génération des recombinants ont été obtenues par traitement d'embryons de poulet pendant 2H à 37°C avec la solution TrypLE™ Select (Gibco) à raison de 200 ml pour 10 embryons. Ces cellules sont ensuite cultivées en milieu MBE (Eagle Based Medium,Gibco) additionné de 5% de SVF, gentamycine 40µg/L à 37°C 5%CO₂

Des boîtes Falcon 3001 sont ensemencées avec 1,5.10⁶ CEPs /boîte dans du milieu MBE additionné de 10% de SVF. Après 48h d'incubation à 37°C 5%CO₂ , les cellules sont infectées avec MVATGN33 à raison de 0,02 ufp/cellule, dilué dans du PBS + cations (acétate de magnésium 100ug/ml, Chlorure de Calcium 100mg/L)+ 1% SVF. Après 30min, 2ml de MBE + 5%SVF sont ajoutés aux cellules infectées. Puis elles sont incubées 1h à 37°C 5% CO₂. Le plasmide de transfert pTG17401 (2 et 5 µg) est précipité dans une solution d'Hepes et de CaCl₂. Le précipité est déposé sur les cellules précédemment infectées. Après 1H à température ambiante, elles sont incubées après adjonction de 2ml de MBE + 5% SVF, à 37°C 5% CO₂ pendant 2h.

Les cellules sont lavées deux fois avec du PBS + cations puis incubées avec 2ml de MBE + 5% SVF à 37°C 5%CO2. Après 48h les boîtes sont congelées.

L'isolement de plaques virales est réalisé en décongelant les boîtes précédentes. Le contenu des boîtes est récupéré dans des tubes Falcon de 6ml. Après sonication, des dilutions sérielles (10⁻¹ à 10⁻³ pour la 1^{ère} sélection et 10⁻³ à 10⁻⁶ pour les sélections suivantes) de ces extraits bruts sont utilisées pour infecter des CEPs.

Une couche de 5ml de milieu MBE gélosé additionné de 5 % SVF d'un mélange d'acide mycophénolique (25µg/ml, Sigma), de xanthine (250µg/ml, Sigma) et d'hypoxanthine (15µg/ml) est déposée dans chaque boîte pour la sélection de virus recombinant GPT+. Les boîtes sont incubées à 37°C 5% CO₂ pendant 72H. Une nouvelle couche de 5ml de milieu MBE gélosé contenant 5% de SVF et un mélange d'acide mycophénolique, de xanthine et d'hypoxanthine et du rouge neutre est déposée sur la couche gélosée précédente. Les boîtes sont ensuite réincubées jusqu'à apparition des plages virales. Les plages virales fluorescentes isolées sont amplifiées sur CEPs puis analysées par PCR pour rechercher la présence du transgène et détecter la contamination par le virus parental MVATGN33. Les clones sélectionnés sont amplifiés sur CEP puis sous-clonés sur milieu sélectif comme décrit ci-dessus, jusqu'à complète élimination du virus sauvage MVATGN33.

Le marqueur de sélection EGFP/GPT a ensuite été éliminé par plusieurs passages sur milieu non-sélectif. Cette élimination est obtenue par recombinaison intragénique entre les séquences homologues encadrant la cassette de sélection.

Après vérification de la présence du gène *gag(dégénéré) pol-nef,* de l'absence du gène EGFP/GPT, et de l'absence de contamination par le virus sauvage MVATGN33, un clone, dénommé ci-après MVATG17401 a été sélectionné pour générer le stock primaire de virus recombinants (dénommé PMVS1). Ce stock est constitué comme suit : 100µl d'amplification du clone choisi sont utilisées pour infecter 2 flasks F175 contenant des CEPs (50ml à DO₅₆₀ₙₘ= 0,23-0,24/ F175) cultivées 48h. Chaque flask contient 25 ml de milieu MBE + 5% SVF. Elles sont incubées pendant 72h à 37°C 5%CO2 puis sont congelées. Après décongélation et sonication, ce stock est titré sur CEP et utilisé comme semence pour la production de virus purifié.

Pour la production du virus recombinant purifié, des flasks F500 ensemencées avec des CEPs cultivées en VP-SFM sont infectées avec le stock primaire à raison de 0,02 ufp/cellule dans du milieu MBE sans sérum pendant 72h à 37°C 5%CO₂. Les cellules infectées sont récoltées après centrifugation puis congelées. Les surnageants de centrifugation sont conservés à 4°C. Le virus est ensuite purifié sur deux coussins consécutifs de sucrose 36% suivi d'un gradient de sucrose (30% à 45%). Les bandes virales sont prélevées et diluées dans du Tris 10mM pH8 au 1/3. Après centrifugation à 4500 rpm pendant 18H, le culot viral est repris dans du tampon puis titré sur CEP.

### EXEMPLE 3: EXPRESSION DE GAG(DEGENERE)-POL-NEF DANS DES FIBROBLASTES EMBRYONNAIRES DE POULET.

Des boîtes de pétri Falcon F3001 sont ensemencées à J-1 avec 1,5.10⁶ CEPs/boîte puis infectées à raison de 0,2 ufp/cellule dans du milieu MBE + 5%SVF et incubées à 37°C 5%CO₂.

Après 24h, le surnageant est éliminé et les cellules sont reprises dans 300µl de Tampon Tris-Glycine 2X (réf: LC2676; Novex) additionné de 5% P-mercaptoéthanol. Le lysat est transféré dans un tube Eppendorf, soniqué et chauffé 5min à 100°C. 10% du matériel est soumis à une électrophorèse sur un gel d'acrylamide (8%) dans des conditions dénaturantes et réductrices dans du Tampon Laemmli.

Les protéines sont ensuite transférées sur membrane PVDF (Macherey Nagel) par électrophorèse à 150mA pendant 16H dans du tampon Tris 25mM, Glycine 192mM, Méthanol 20%. Les membranes sont saturées dans du tampon de saturation PBS 1X, Tween 20 0,5%, SVF 5% pendant 2H.

Les membranes sont ensuite mises pendant 2H en présence de l'anticorps primaire (sérum polyclonal issu d'un pool de patients immunisés avec un mélange de fragments peptidiques de VIH, contenant des peptides homologues aux fragments 66-97, 117-147 et 182-205 de la protéine Nef, aux fragments 183-214 et 253-284 de la protéine Gag, et au fragment 303-335 de la protéine Env), dilué au 1/3000^{ème} dans le tampon de saturation. Puis les membranes sont lavées dans du tampon de lavage PBS1X trois fois pendant 10min. Les membranes sont ensuite mises en présence de l'anticorps secondaire, anticorps anti-IgG humaines biotinylé (Amersham) dilué au 1/500^{e} dans le tampon de saturation pendant 1H. Elles sont lavées comme précédemment. Les membranes sont enfin mises pendant 30min en présence d'un complexe streptavidine-péroxydase biotinylée (Amersham) dilué dans le tampon de saturation au 1/1000^{e}. La révélation se fait avec le Kit ECL (Enhanced Chemiluminescence, Amersham).

Les résultats sont illustrés par la Figure 4.

Malgré la faible spécificité du sérum polyclonal humain utilisé, qui reconnaît une protéine de MVATGN33 qui migre quasiment au même niveau que la protéine de fusion Gag-Pol-Nef, ces résultats montrent que la cassette d'expression de *gag(dégénéré) pol-nef* contenue dans le virus MVATG17401 est fonctionnelle. La protéine de fusion exprimée à partir de ce vecteur se situe à environ 100 kDa (97kDa théorique), qui correspond au poids moléculaire attendu pour la protéine de fusion Gag-Pol-Nef.

Un séquençage de l'insert dans le virus MVATG17401 contenant l'ORF codant la protéine de fusion Gag-Pol-Nef a montré que la protéine de fusion est constituée de 795 acides aminés et est identique à la séquence théorique attendue.

### EXEMPLE 4: STABILITE GENETIQUE DU VECTEUR RECOMBINANT MVATG17401 A DES NIVEAUX DE PASSAGE EQUIVALENTS AU LOT CLINIQUE.

La stabilité génétique du vecteur recombinant MVATG17401 à un niveau de passage équivalent au lot clinique a été évaluée. Pour cela, un lot de qualité BPF, directement dérivé du lot de semence primaire (LSP), a été analysé par PCR, Western blot et séquençage. Plus de 100 plaques individuelles isolées à partir du même matériel ont été analysées par Western blot pour vérifier la stabilité génétique du vecteur recombinant MVATG17401.

### Matériels et méthodes

### Matériel BPF

L'expression de la protéine de fusion a été analysée par Western blot pour le LSP (lot Y501), le matériel vrac du lot toxicologique avant remplissage (X511E03) et du lot clinique (Z568). La filiation des lots est montrée sur la Figure 5. L'analyse PCR et le séquençage ont été effectués sur l'Adn isolé du lot de toxicologie après remplissage (Y511). L'analyse de l'expression de la protéine de fusion par Western blot a aussi été mise en oeuvre sur des clones individuels isolés du matériel vrac du lot toxicologique (X511E03).

### Caractérisation de l'insert génétique par PCR

Deux paires d'amorces ont été utilisées pour couvrir la totalité de la cassette d'expression et les séquences virales flanquantes. La taille des fragments amplifiés est de 1682 pb (fragment PCR A, amorce sens : CATGACGAGCTTCCGAGTTC (SEQ ID NO :5), amorce antisens : GTTGAAGCACTTCACCATCTTCCTCTG (SEQ ID NO :6)) et 1833 pb (fragment PCR B, amorce sens : CCTGAACAAGATCGTGAGGATG (SEQ ID NO :7), amorce antisens : GCTCCTTATACCAAGCACTC (SEQ ID NO :8)).

### Western blot

Des cellules A549 infectées ont été lysées avec du tampon LDS en présence de β-mercaptoéthanol. Les protéines ont été séparées par éléctrophorèse sur gel de polyacrylamide 10% et transférées sur une membrane PVDF. La polyprotéine a été détectée avec un anticorps monoclonal murin dirigé contre la protéine Gag du VIH-1 (anti-VIH-1-p24). Des anticorps de chèvre anti-anticorps murins liés à la biotine et un complexe streptavidine-péroxydase ont été utilisés pour le marquage.

### Sous-clonage et détermination de la proportion de clones exprimant la protéine de fusion

Des plaques du vecteur MVATG17401 ont été isolées à partir de couches de cellules BHK infectées avec le matériel vrac du lot toxicologique (X511E03) et recouvertes d'agarose. Un total de 113 plaques a été isolé et amplifié en deux cycles successifs dans des plaques 96 puits. Une plaque suspectée d'être contaminée a été écartée de la suite de l'analyse. Les autres plaques ont été utilisées pour infecter des cellules A549 de manière à exprimer la protéine de fusion.

### Spécificité des anticorps monoclonaux utilisés pour l'immunodétection

L'anticorps monoclonal murin anti-VIH-1-p24 (1A) provient de Perkin Elmer (ref NEA-9306). Il a été produit contre un lysat purifié de la souche HTLV-111 B du HIV-1. Cet anticorps est spécifique de la protéine structurale du coeur p24 codée par gag et du précurseur Gag p55.

### Résultats

La caractérisation de l'insert génétique sur l'ADN isolé du lot toxicologique (Y511) montre que la taille des fragments A et B amplifiés par PCR et la séquence de l'insert sont conformes aux attentes.

La proportion de virus qui expriment la protéine de fusion a été déterminée sur un échantillon du lot toxicologique avant remplissage (X511E03) et a montré que 100% des clones expriment la protéine de fusion.

En outre, l'expression de la protéine de fusion a été évaluée dans des échantillons des lots de semence primaire (LSP), lot toxicologique (X511E03) et lot clinique (Z568). La protéine de fusion a été détectée dans tous les échantillons, sans qu'aucune différence ne soit détectée avec l'échantillon témoin positif alors que la protéine de fusion était indétectable dans les échantillons contrôle négatifs.

Ces résultats montrent que le vecteur MVATG17401 est stable génétiquement au niveau de passage du lot clinique Z568 ce qui valide donc l'utilisation du lot de semence primaire pour la production de lots cliniques au même niveau de passage que Z568.

## Revendications

1. Polynucléotide codant pour la protéine Gag (p55) de VIH-1, **caractérisé en ce qu'**il est défini par la séquence SEQ ID NO: 1.

2. Polynucléotide recombinant codant pour une protéine de fusion comprenant la protéine Gag (p55) de VIH-1, fusionnée avec un ou plusieurs autre(s) polypeptide (s) de VIH, **caractérisé en ce qu'**il comprend la séquence SEQ ID NO: 1.

3. Polynucléotide recombinant selon la revendication 2, **caractérisé en ce que** ledit ou lesdits autre(s) polypeptide (s) de VIH est (sont) choisi(s) parmi la protéine Pol, la protéine Nef, la protéine Env, ou tout fragment desdites protéines portant au moins un épitope reconnu par des anticorps anti-VIH, ou par des lymphocytes T cytotoxiques CTLs anti-VIH.

4. Polynucléotide recombinant selon la revendication 3, **caractérisé en ce qu'**il est défini par la séquence SEQ ID NO: 2.

5. Vecteur recombinant comprenant un polynucléotide selon une quelconque des revendications 1 à 4.

6. Vecteur recombinant selon la revendication 5, **caractérisé en ce qu'**il s'agit d'un poxvirus atténué.

7. Vecteur recombinant selon la revendication 6, **caractérisé en ce que** ledit poxvirus est le virus Ankara modifié (MVA).

8. Utilisation d'un vecteur recombinant selon une quelconque des revendications 5 à 7, pour l'obtention d'un vaccin anti-VIH.

9. Composition immunogène comprenant un vecteur recombinant selon l'une quelconque des revendications 5 à 7.

10. Composition vaccinale comprenant un vecteur recombinant selon l'une quelconque des revendications 5 à 7.

11. Vecteur recombinant selon l'une quelconque des revendications 5 à 7 pour son utilisation pour induire une réponse immune chez un sujet par son administration à un sujet qui le nécessite.

12. Vecteur recombinant selon l'une quelconque des revendications 5 à 7 pour son utilisation pour la prévention ou le traitement d'une infection par le VIH par son administration à un sujet qui le nécessite.

## Patentansprüche

1. Polynukleotid, das für das Protein Gag (p55) von HIV-1 kodiert, **dadurch gekennzeichnet, dass** es durch die Sequenz SEQ ID NO: 1 definiert ist.

2. Rekombinantes Polynukleotid, das für ein Fusionsprotein, umfassend das Protein Gag (p55) von HIV-1, kodiert, fusioniert mit einem oder mehreren anderen Polypeptid(en) von HIV, **dadurch gekennzeichnet, dass** es die Sequenz SEQ ID NO: 1 umfasst.

3. Rekombinantes Polynukleotid gemäß Anspruch 2, **dadurch gekennzeichnet, dass** der oder die andere(n) Polypeptid(e) von HIV ausgewählt ist (sind) aus dem Protein Pol, dem Protein Nef, dem Protein Env, oder jedem Fragment der Proteine, welches mindestens ein Epitop trägt, welches von den Antikörpern anti-HIV oder von den cytotoxischen T-Lymphozyten CTLs anti-HIV erkannt wird.

4. Rekombinantes Polynukleotid gemäß Anspruch 3, **dadurch gekennzeichnet, dass** es durch die Sequenz SEQ ID NO: 2 definiert ist.

5. Rekombinanter Vektor, welcher ein Polynukleotid gemäß einem der Ansprüche 1 bis 4 umfasst.

6. Rekombinanter Vektor gemäß Anspruch 5, **dadurch gekennzeichnet, dass** es sich um einen abgeschwächten Pockenvirus handelt.

7. Rekombinanter Vektor gemäß Anspruch 6, **dadurch gekennzeichnet dass** der Pockenvirus der modifizierte Ankara Virus (MVA) ist.

8. Verwendung eines rekombinanten Vektors gemäß einem der Ansprüche 5 bis 7, zum Erhalt eines HIV-Impfstoffes.

9. Immunogene Zusammensetzung, welche einen rekombinanten Vektor gemäß einem der Ansprüche 5 bis 7 umfasst.

10. Impfstoffzusammensetzung, welche einen rekombinanten Vektor gemäß einem der Ansprüche 5 bis 7 umfasst.

11. Rekombinanter Vektor gemäß einem der Ansprüche 5 bis 7 zur Verwendung um eine Immunreaktion in einem Testsubjekt hervorzurufen durch Verabreichung an ein Testsubjekt das einer solchen bedarf.

12. Rekombinanter Vektor gemäß einem der Ansprüche 5 bis 7 zur Verwendung für die Vorbeugung oder die Behandlung einer HIV-Infektion durch Verabreichung an ein Testsubjekt das einer solchen bedarf.

## Claims

1. A polynucleotide coding for the HIV-1 Gag (p55) protein, **characterized in that** it is defined by the sequence SEQ ID NO: 1.

2. A recombinant polynucleotide coding for a fusion protein comprising the HIV-1 Gag (p55) protein, fused with one or more other HIV polypeptide(s), **characterized in that** it comprises the sequence SEQ ID NO: 1.

3. The recombinant polynucleotide according to claim 2, **characterized in that** said other HIV polypeptide(s) is(are) selected from the Pol protein, the Nef protein, the Env protein, or any fragment of said proteins bearing at least one epitope recognized by anti-HIV antibodies, or by anti-HIV cytotoxic T lymphocytes (CTLs).

4. The recombinant polynucleotide according to claim 3, **characterized in that** it is defined by the sequence SEQ ID NO: 2.

5. A recombinant vector comprising a polynucleotide according to any one of claims 1 to 4.

6. The recombinant vector according to claim 5, **characterized in that** it is an attenuated poxvirus.

7. The recombinant vector according to claim 6, **characterized in that** said poxvirus is the modified Ankara virus (MVA).

8. The use of a recombinant vector according to any one of claims 5 to 7, for obtaining an anti-HIV vaccine.

9. An immunogenic composition comprising a recombinant vector according to any one of claims 5 to 7.

10. A vaccinal composition comprising a recombinant vector according to any one of claims 5 to 7.

11. The recombinant vector according to any one of claims 5 to 7 for use for inducing an immune response in a subject by its administration to a subject requiring it.

12. The recombinant vector according to any one of claims 5 to 7 for use in the prevention or treatment of an HIV infection by its administration to a subject requiring it.


### SEQUENCE LISTING

<110> INSERM
<120> Synthetic gene coding HIV-1 GAG protein
<130> BET 09P1156
<150> EP 08290849.2
   <151> 2008-09-10
<160> 8
<170> PatentIn version 3.5
<210> 1
   <211> 1543
   <212> DNA
   <213> Artificial
<220>
   <223> synthetic GAG Gene
<400> 1
   atgggcgcca gggccagcgt gctgagcgga ggcgagctgg acaggtggga gaagatcagg      60
   ctgaggcctg gaggcaagaa gaagtataag ctgaagcaca tcgtgtgggc cagcagggag     120
   ctggagaggt tcgccgtgaa ccctggcctg ctggagacca gcgagggctg caggcagatc     180
   ctgggccagc tgcagcccag cctgcagacc ggcagcgagg agctgaggag cctgtacaac     240
   accgtggcca ccctgtactg cgtgcaccag aggatcgaga tcaaggacac caaggaggcc     300
   ctggacaaga tcgaggagga gcagaacaag tccaagaaga aggcccagca ggctgctgcc     360
   gacaccggcc acagcagcca ggtgagccag aactacccta tcgtgcagaa catccagggc     420
   cagatggtgc accaggccat cagccctagg accctgaacg cctgggtgaa ggtggtggag     480
   gagaaggcct tcagccctga ggtgatccct atgttcagcg ccctgagcga gggagccaca     540
   cctcaggacc tgaacaccat gctgaacacc gtgggaggcc accaggccgc catgcagatg     600
   ctgaaggaga ccatcaacga ggaggctgcc gagtgggaca gggtgcaccc tgtgcacgct     660
   ggacccatcg ctccaggcca gatgagggag cccagaggca gcgacatcgc cggcaccacc     720
   agcaccctgc aggagcagat cggctggatg accaacaacc ctcccatccc tgtgggcgaa     780
   atctacaaga ggtggatcat cctgggcctg aacaagatcg tgaggatgta cagccctacc     840
   agcatcctgg atatcaggca gggccctaaa gagcccttca gggactacgt ggacaggttc     900
   tacaagaccc tgagagccga gcaggccagc caggaggtga agaactggat gaccgagacc     960
   ctgctggtgc agaacgccaa ccctgactgc aagaccatcc tgaaggccct gggacctgct    1020
   gccaccctgg aggagatgat gaccgcctgc cagggcgtgg gaggcccagg ccacaaggcc    1080
   agggtgctgg ccgaggccat gagccaggtg accaacaccg ccaccatcat gatgcagaga    1140
   ggcaacttca ggaaccagag gaagatggtg aagtgcttca actgcggcaa ggagggccac    1200
   accgccagga actgcagggc tcccaggaag aagggctgct ggaagtgcgg caaggagggc    1260
   caccagatga aggactgcac cgagaggcag gccaacttcc tgggcaagat ctggcccagc    1320
   tacaagggca ggccaggcaa cttcctgcag agcaggcccg agcccaccgc tccacctttc    1380
   ctgcagagca ggcccgagcc caccgctcct cctgaggaga gcttcaggag cggcgtggag    1440
   acaaccaccc ctcctcagaa gcaggagccc atcgacaagg agctgtaccc tctgaccagc    1500
   ctgaggagcc tgttcggcaa cgaccctagc agccaggagt cga                      1543
<210> 2
   <211> 2388
   <212> DNA
   <213> Artificial
<220>
   <223> synthetic gag -nef-pol
<220>
   <221> CDS
   <222> (1)..(2388)
<400> 2
   atg ggc gcc agg gcc agc gtg ctg agc gga ggc gag ctg gac agg tgg          48
   Met Gly Ala Arg Ala Ser Val Leu Ser Gly Gly Glu Leu Asp Arg Trp
   1               5                   10                  15
   gag aag atc agg ctg agg cct gga ggc aag aag aag tat aag ctg aag           96
   Glu Lys Ile Arg Leu Arg Pro Gly Gly Lys Lys Lys Tyr Lys Leu Lys
               20                  25                  30
   cac atc gtg tgg gcc agc agg gag ctg gag agg ttc gcc gtg aac cct          144
   His Ile Val Trp Ala Ser Arg Glu Leu Glu Arg Phe Ala Val Asn Pro
           35                  40                  45
   ggc ctg ctg gag acc agc gag ggc tgc agg cag atc ctg ggc cag ctg          192
   Gly Leu Leu Glu Thr Ser Glu Gly Cys Arg Gln Ile Leu Gly Gln Leu
       50                  55                  60
   cag ccc agc ctg cag acc ggc agc gag gag ctg agg agc ctg tac aac          240
   Gln Pro Ser Leu Gln Thr Gly Ser Glu Glu Leu Arg Ser Leu Tyr Asn
   65                  70                  75                  80
   acc gtg gcc acc ctg tac tgc gtg cac cag agg atc gag atc aag gac          288
   Thr Val Ala Thr Leu Tyr Cys Val His Gln Arg Ile Glu Ile Lys Asp
                   85                  90                  95
   acc aag gag gcc ctg gac aag atc gag gag gag cag aac aag tcc aag          336
   Thr Lys Glu Ala Leu Asp Lys Ile Glu Glu Glu Gln Asn Lys Ser Lys
               100                 105                 110
   aag aag gcc cag cag gct gct gcc gac acc ggc cac agc agc cag gtg          384
   Lys Lys Ala Gln Gln Ala Ala Ala Asp Thr Gly His Ser Ser Gln Val
           115                 120                 125
   agc cag aac tac cct atc gtg cag aac atc cag ggc cag atg gtg cac          432
   Ser Gln Asn Tyr Pro Ile Val Gln Asn Ile Gln Gly Gln Met Val His
       130                 135                 140
   cag gcc atc agc cct agg acc ctg aac gcc tgg gtg aag gtg gtg gag          480
   Gln Ala Ile Ser Pro Arg Thr Leu Asn Ala Trp Val Lys Val Val Glu
   145                 150                 155                 160
   gag aag gcc ttc agc cct gag gtg atc cct atg ttc agc gcc ctg agc          528
   Glu Lys Ala Phe Ser Pro Glu Val Ile Pro Met Phe Ser Ala Leu Ser
                   165                 170                 175
   gag gga gcc aca cct cag gac ctg aac acc atg ctg aac acc gtg gga          576
   Glu Gly Ala Thr Pro Gln Asp Leu Asn Thr Met Leu Asn Thr Val Gly
               180                 185                 190
   ggc cac cag gcc gcc atg cag atg ctg aag gag acc atc aac gag gag          624
   Gly His Gln Ala Ala Met Gln Met Leu Lys Glu Thr Ile Asn Glu Glu
           195                 200                 205
   gct gcc gag tgg gac agg gtg cac cct gtg cac gct gga ccc atc gct          672
   Ala Ala Glu Trp Asp Arg Val His Pro Val His Ala Gly Pro Ile Ala
       210                 215                 220
   cca ggc cag atg agg gag ccc aga ggc agc gac atc gcc ggc acc acc          720
   Pro Gly Gln Met Arg Glu Pro Arg Gly Ser Asp Ile Ala Gly Thr Thr
   225                 230                 235                 240
   agc acc ctg cag gag cag atc ggc tgg atg acc aac aac cct ccc atc          768
   Ser Thr Leu Gln Glu Gln Ile Gly Trp Met Thr Asn Asn Pro Pro Ile
                   245                 250                 255
   cct gtg ggc gaa atc tac aag agg tgg atc atc ctg ggc ctg aac aag          816
   Pro Val Gly Glu Ile Tyr Lys Arg Trp Ile Ile Leu Gly Leu Asn Lys
               260                 265                 270
   atc gtg agg atg tac agc cct acc agc atc ctg gat atc agg cag ggc          864
   Ile Val Arg Met Tyr Ser Pro Thr Ser Ile Leu Asp Ile Arg Gln Gly
           275                 280                 285
   cct aaa gag ccc ttc agg gac tac gtg gac agg ttc tac aag acc ctg          912
   Pro Lys Glu Pro Phe Arg Asp Tyr Val Asp Arg Phe Tyr Lys Thr Leu
       290                 295                 300
   aga gcc gag cag gcc agc cag gag gtg aag aac tgg atg acc gag acc          960
   Arg Ala Glu Gln Ala Ser Gln Glu Val Lys Asn Trp Met Thr Glu Thr
   305                 310                 315                 320
   ctg ctg gtg cag aac gcc aac cct gac tgc aag acc atc ctg aag gcc         1008
   Leu Leu Val Gln Asn Ala Asn Pro Asp Cys Lys Thr Ile Leu Lys Ala
                   325                 330                 335
   ctg gga cct gct gcc acc ctg gag gag atg atg acc gcc tgc cag ggc        1056
   Leu Gly Pro Ala Ala Thr Leu Glu Glu Met Met Thr Ala Cys Gln Gly
               340                 345                 350
   gtg gga ggc cca ggc cac aag gcc agg gtg ctg gcc gag gcc atg agc        1104
   Val Gly Gly Pro Gly His Lys Ala Arg Val Leu Ala Glu Ala Met Ser
           355                 360                 365
   cag gtg acc aac acc gcc acc atc atg atg cag aga ggc aac ttc agg        1152
   Gln Val Thr Asn Thr Ala Thr Ile Met Met Gln Arg Gly Asn Phe Arg
       370                 375                 380
   aac cag agg aag atg gtg aag tgc ttc aac tgc ggc aag gag ggc cac        1200
   Asn Gln Arg Lys Met Val Lys Cys Phe Asn Cys Gly Lys Glu Gly His
   385                 390                 395                 400
   acc gcc agg aac tgc agg gct ccc agg aag aag ggc tgc tgg aag tgc        1248
   Thr Ala Arg Asn Cys Arg Ala Pro Arg Lys Lys Gly Cys Trp Lys Cys
                   405                 410                 415
   ggc aag gag ggc cac cag atg aag gac tgc acc gag agg cag gcc aac        1296
   Gly Lys Glu Gly His Gln Met Lys Asp Cys Thr Glu Arg Gln Ala Asn
               420                 425                 430
   ttc ctg ggc aag atc tgg ccc agc tac aag ggc agg cca ggc aac ttc        1344
   Phe Leu Gly Lys Ile Trp Pro Ser Tyr Lys Gly Arg Pro Gly Asn Phe
           435                 440                 445
   ctg cag agc agg ccc gag ccc acc gct cca cct ttc ctg cag agc agg        1392
   Leu Gln Ser Arg Pro Glu Pro Thr Ala Pro Pro Phe Leu Gln Ser Arg
       450                 455                 460
   ccc gag ccc acc gct cct cct gag gag agc ttc agg agc ggc gtg gag        1440
   Pro Glu Pro Thr Ala Pro Pro Glu Glu Ser Phe Arg Ser Gly Val Glu
   465                 470                 475                 480
   aca acc acc cct cct cag aag cag gag ccc atc gac aag gag ctg tac        1488
   Thr Thr Thr Pro Pro Gln Lys Gln Glu Pro Ile Asp Lys Glu Leu Tyr
                   485                 490                 495
   cct ctg acc agc ctg agg agc ctg ttc ggc aac gac cct agc agc cag        1536
   Pro Leu Thr Ser Leu Arg Ser Leu Phe Gly Asn Asp Pro Ser Ser Gln
               500                 505                 510
   gag tcg acc ggg cca cta aca gaa gaa gca gag cta gaa ctg gca gaa        1584
   Glu Ser Thr Gly Pro Leu Thr Glu Glu Ala Glu Leu Glu Leu Ala Glu
           515                 520                 525
   aac aga gag att cta aaa gaa cca gta cat gga gtg tat tat gac cca        1632
   Asn Arg Glu Ile Leu Lys Glu Pro Val His Gly Val Tyr Tyr Asp Pro
       530                 535                 540
   tca aaa gac tta ata gca gaa ata cag aag cag ggg caa ggc caa tgg        1680
   Ser Lys Asp Leu Ile Ala Glu Ile Gln Lys Gln Gly Gln Gly Gln Trp
   545                 550                 555                 560
   aca tat caa att tat caa gag cca ttt aaa aat ctg aaa aca gga atg        1728
   Thr Tyr Gln Ile Tyr Gln Glu Pro Phe Lys Asn Leu Lys Thr Gly Met
                   565                 570                 575
   gag tgg aga ttt gat tct aga tta gca ttt cat cac gta gct aga gaa        1776
   Glu Trp Arg Phe Asp Ser Arg Leu Ala Phe His His Val Ala Arg Glu
               580                 585                 590
   tta cat cct gaa tat ttt aaa aat tgt aag ctt atg gca ata ttc caa        1824
   Leu His Pro Glu Tyr Phe Lys Asn Cys Lys Leu Met Ala Ile Phe Gln
           595                 600                 605
   agt agc atg aca aaa atc tta gag cct ttt aga aaa caa aat cca gac        1872
   Ser Ser Met Thr Lys Ile Leu Glu Pro Phe Arg Lys Gln Asn Pro Asp
       610                 615                 620
   ata gtt atc tat caa tac atg gat gat ttg tat gta gga tct gac tta        1920
   Ile Val Ile Tyr Gln Tyr Met Asp Asp Leu Tyr Val Gly Ser Asp Leu
   625                 630                 635                 640
   gaa ata ggg cag cat aga aca aaa ata gag gag ctg aga caa cat ctg        1968
   Glu Ile Gly Gln His Arg Thr Lys Ile Glu Glu Leu Arg Gln His Leu
                   645                 650                 655
   ttg agg tgg gga ctt aca acc atg gta ggt ttt cca gta aca cct caa        2016
   Leu Arg Trp Gly Leu Thr Thr Met Val Gly Phe Pro Val Thr Pro Gln
               660                 665                 670
   gta cct tta aga cca atg act tac aaa gca gct gta gat ctt tct cac        2064
   Val Pro Leu Arg Pro Met Thr Tyr Lys Ala Ala Val Asp Leu Ser His
           675                 680                 685
   ttt tta aaa gaa aaa gga ggt tta gaa ggg cta att cat tct caa cga        2112
   Phe Leu Lys Glu Lys Gly Gly Leu Glu Gly Leu Ile His Ser Gln Arg
       690                 695                 700
   aga caa gat att ctt gat ttg tgg att tat cat aca caa gga tat ttt        2160
   Arg Gln Asp Ile Leu Asp Leu Trp Ile Tyr His Thr Gln Gly Tyr Phe
   705                 710                 715                 720
   cct gat tgg cag aat tac aca cca gga cca gga gtc aga tac cca tta        2208
   Pro Asp Trp Gln Asn Tyr Thr Pro Gly Pro Gly Val Arg Tyr Pro Leu
                   725                 730                 735
   acc ttt ggt tgg tgc tac aag cta gta cca atg att gag act gta cca        2256
   Thr Phe Gly Trp Cys Tyr Lys Leu Val Pro Met Ile Glu Thr Val Pro
               740                 745                 750
   gta aaa tta aag cca gga atg gat ggc cca aaa gtt aaa caa tgg cca        2304
   Val Lys Leu Lys Pro Gly Met Asp Gly Pro Lys Val Lys Gln Trp Pro
           755                 760                 765
   ttg aca gaa gaa aaa ata aaa gca tta gta gaa att tgt aca gag atg        2352
   Leu Thr Glu Glu Lys Ile Lys Ala Leu Val Glu Ile Cys Thr Glu Met
       770                 775                 780
   gaa aag gaa ggg aaa att tca aaa att ggg cct taa                        2388
   Glu Lys Glu Gly Lys Ile Ser Lys Ile Gly Pro
   785                 790                 795
<210> 3
   <211> 795
   <212> PRT
   <213> Artificial
<220>
   <223> Synthetic Construct
<400> 3
   Met Gly Ala Arg Ala Ser Val Leu Ser Gly Gly Glu Leu Asp Arg Trp
   1               5                   10                  15
   Glu Lys Ile Arg Leu Arg Pro Gly Gly Lys Lys Lys Tyr Lys Leu Lys
               20                  25                  30
   His Ile Val Trp Ala Ser Arg Glu Leu Glu Arg Phe Ala Val Asn Pro
           35                  40                  45
   Gly Leu Leu Glu Thr Ser Glu Gly Cys Arg Gln Ile Leu Gly Gln Leu
       50                  55                  60
   Gln Pro Ser Leu Gln Thr Gly Ser Glu Glu Leu Arg Ser Leu Tyr Asn
   65                  70                  75                  80
   Thr Val Ala Thr Leu Tyr Cys Val His Gln Arg Ile Glu Ile Lys Asp
                   85                  90                  95
   Thr Lys Glu Ala Leu Asp Lys Ile Glu Glu Glu Gln Asn Lys Ser Lys
               100                 105                 110
   Lys Lys Ala Gln Gln Ala Ala Ala Asp Thr Gly His Ser Ser Gln Val
           115                 120                 125
   Ser Gln Asn Tyr Pro Ile Val Gln Asn Ile Gln Gly Gln Met Val His
       130                 135                 140
   Gln Ala Ile Ser Pro Arg Thr Leu Asn Ala Trp Val Lys Val Val Glu
   145                 150                 155                 160
   Glu Lys Ala Phe Ser Pro Glu Val Ile Pro Met Phe Ser Ala Leu Ser
                   165                 170                 175
   Glu Gly Ala Thr Pro Gln Asp Leu Asn Thr Met Leu Asn Thr Val Gly
               180                 185                 190
   Gly His Gln Ala Ala Met Gln Met Leu Lys Glu Thr Ile Asn Glu Glu
           195                 200                 205
   Ala Ala Glu Trp Asp Arg Val His Pro Val His Ala Gly Pro Ile Ala
       210                 215                 220
   Pro Gly Gln Met Arg Glu Pro Arg Gly Ser Asp Ile Ala Gly Thr Thr
   225                 230                 235                 240
   Ser Thr Leu Gln Glu Gln Ile Gly Trp Met Thr Asn Asn Pro Pro Ile
                   245                 250                 255
   Pro Val Gly Glu Ile Tyr Lys Arg Trp Ile Ile Leu Gly Leu Asn Lys
               260                 265                 270
   Ile Val Arg Met Tyr Ser Pro Thr Ser Ile Leu Asp Ile Arg Gln Gly
           275                 280                 285
   Pro Lys Glu Pro Phe Arg Asp Tyr Val Asp Arg Phe Tyr Lys Thr Leu
       290                 295                 300
   Arg Ala Glu Gln Ala Ser Gln Glu Val Lys Asn Trp Met Thr Glu Thr
   305                 310                 315                 320
   Leu Leu Val Gln Asn Ala Asn Pro Asp Cys Lys Thr Ile Leu Lys Ala
                   325                 330                 335
   Leu Gly Pro Ala Ala Thr Leu Glu Glu Met Met Thr Ala Cys Gln Gly
               340                 345                 350
   Val Gly Gly Pro Gly His Lys Ala Arg Val Leu Ala Glu Ala Met Ser
           355                 360                 365
   Gln Val Thr Asn Thr Ala Thr Ile Met Met Gln Arg Gly Asn Phe Arg
       370                 375                 380
   Asn Gln Arg Lys Met Val Lys Cys Phe Asn Cys Gly Lys Glu Gly His
   385                 390                 395                 400
   Thr Ala Arg Asn Cys Arg Ala Pro Arg Lys Lys Gly Cys Trp Lys Cys
                   405                 410                 415
   Gly Lys Glu Gly His Gln Met Lys Asp Cys Thr Glu Arg Gln Ala Asn
               420                 425                 430
   Phe Leu Gly Lys Ile Trp Pro Ser Tyr Lys Gly Arg Pro Gly Asn Phe
           435                 440                 445
   Leu Gln Ser Arg Pro Glu Pro Thr Ala Pro Pro Phe Leu Gln Ser Arg
       450                 455                 460
   Pro Glu Pro Thr Ala Pro Pro Glu Glu Ser Phe Arg Ser Gly Val Glu
   465                 470                 475                 480
   Thr Thr Thr Pro Pro Gln Lys Gln Glu Pro Ile Asp Lys Glu Leu Tyr
                   485                 490                 495
   Pro Leu Thr Ser Leu Arg Ser Leu Phe Gly Asn Asp Pro Ser Ser Gln
               500                 505                 510
   Glu Ser Thr Gly Pro Leu Thr Glu Glu Ala Glu Leu Glu Leu Ala Glu
           515                 520                 525
   Asn Arg Glu Ile Leu Lys Glu Pro Val His Gly Val Tyr Tyr Asp Pro
       530                 535                 540
   Ser Lys Asp Leu Ile Ala Glu Ile Gln Lys Gln Gly Gln Gly Gln Trp
   545                 550                 555                 560
   Thr Tyr Gln Ile Tyr Gln Glu Pro Phe Lys Asn Leu Lys Thr Gly Met
                   565                 570                 575
   Glu Trp Arg Phe Asp Ser Arg Leu Ala Phe His His Val Ala Arg Glu
               580                 585                 590
   Leu His Pro Glu Tyr Phe Lys Asn Cys Lys Leu Met Ala Ile Phe Gln
           595                 600                 605
   Ser Ser Met Thr Lys Ile Leu Glu Pro Phe Arg Lys Gln Asn Pro Asp
       610                 615                 620
   Ile Val Ile Tyr Gln Tyr Met Asp Asp Leu Tyr Val Gly Ser Asp Leu
   625                 630                 635                 640
   Glu Ile Gly Gln His Arg Thr Lys Ile Glu Glu Leu Arg Gln His Leu
                   645                 650                 655
   Leu Arg Trp Gly Leu Thr Thr Met Val Gly Phe Pro Val Thr Pro Gln
               660                 665                 670
   Val Pro Leu Arg Pro Met Thr Tyr Lys Ala Ala Val Asp Leu Ser His
           675                 680                 685
   Phe Leu Lys Glu Lys Gly Gly Leu Glu Gly Leu Ile His Ser Gln Arg
       690                 695                 700
   Arg Gln Asp Ile Leu Asp Leu Trp Ile Tyr His Thr Gln Gly Tyr Phe
   705                 710                 715                 720
   Pro Asp Trp Gln Asn Tyr Thr Pro Gly Pro Gly Val Arg Tyr Pro Leu
                   725                 730                 735
   Thr Phe Gly Trp Cys Tyr Lys Leu Val Pro Met Ile Glu Thr Val Pro
               740                 745                 750
   Val Lys Leu Lys Pro Gly Met Asp Gly Pro Lys Val Lys Gln Trp Pro
           755                 760                 765
   Leu Thr Glu Glu Lys Ile Lys Ala Leu Val Glu Ile Cys Thr Glu Met
       770                 775                 780
   Glu Lys Glu Gly Lys Ile Ser Lys Ile Gly Pro
   785                 790                 795
<210> 4
   <211> 6000
   <212> DNA
   <213> artificial
<220>
   <223> Fragment of vector pTG17401
<400> 4
   aaataaatca tataaaaaat gatttcatga ttaaaccatg ttgtgaaaaa gtcaagaacg      60
   ttcacattgg cggacaatct aaaaacaata cagtgattgc agatttgcca tatatggata     120
   atgcggtatc cgatgtatgc aattcactgt ataaaaagaa tgtatcaaga atatccagat     180
   ttgctaattt gataaagata gatgacgatg acaagactcc tactggtgta tataattatt     240
   ttaaacctaa agatgccatt cctgttatta tatccatagg aaaggataga gatgtttgtg     300
   aactattaat ctcatctgat aaagcgtgtg cgtgtataga gttaaattca tataaagtag     360
   ccattcttcc catggatgtt tcctttttta ccaaaggaaa tgcatcattg attattctcc     420
   tgtttgattt ctctatcgat gcggcacctc tcttaagaag tgtaaccgat aataatgtta     480
   ttatatctag acaccagcgt ctacatgacg agcttccgag ttccaattgg ttcaagtttt     540
   acataagtat aaagtccgac tattgttcta tattatatat ggttgttgat ggatctgtga     600
   tgcatgcaat agctgataat agaacttacg caaatattag caaaaatata ttagacaata     660
   ctacaattaa cgatgagtgt agatgctgtt attttgaacc acagattagg attcttgata     720
   gagatgagat gctcaatgga tcatcgtgtg atatgaacag acattgtatt atgatgaatt     780
   tacctgatgt aggcgaattt ggatctagta tgttggggaa atatgaacct gacatgatta     840
   agattgctct ttcggtggct ggtgagctcg gatctaagct tgtcgacata aaaatatagt     900
   agaatttcat ttgttttttt ctatgctata aataggatcg atccgataaa gtgaaaaata     960
   attctaattt attgcacggt aaggaagtag aatcataaag aaaagcttct gcaggtcgac    1020
   atggtgagca agggcgagga gctgttcacc ggggtggtgc ccatcctggt cgagctggac    1080
   ggcgacgtaa acggccacaa gttcagcgtg tccggcgagg gcgagggcga tgccacctac    1140
   ggcaagctga ccctgaagtt catctgcacc accggcaagc tgcccgtgcc ctggcccacc    1200
   ctcgtgacca ccctgaccta cggcgtgcag tgcttcagcc gctaccccga ccacatgaag    1260
   cagcacgact tcttcaagtc cgccatgccc gaaggctacg tccaggagcg caccatcttc    1320
   ttcaaggacg acggcaacta caagacccgc gccgaggtga agttcgaggg cgacaccctg    1380
   gtgaaccgca tcgagctgaa gggcatcgac ttcaaggagg acggcaacat cctggggcac    1440
   aagctggagt acaactacaa cagccacaac gtctatatca tggccgacaa gcagaagaac    1500
   ggcatcaagg tgaacttcaa gatccgccac aacatcgagg acggcagcgt gcagctcgcc    1560
   gaccactacc agcagaacac ccccatcggc gacggccccg tgctgctgcc cgacaaccac    1620
   tacctgagca cccagtccgc cctgagcaaa gaccccaacg agaagcgcga tcacatggtc    1680
   ctgctggagt tcgtgaccgc cgccgggatc actctcggca tggacgagct gtacaagagc    1740
   gaaaaataca tcgtcacctg ggacatgttg cagatccatg cacgtaaact cgcaagccga    1800
   ctgatgcctt ctgaacaatg gaaaggcatt attgccgtaa gccgtggcgg tctggtaccg    1860
   ggtgcgttac tggcgcgtga actgggtatt cgtcatgtcg ataccgtttg tatttccagc    1920
   tacgatcacg acaaccagcg cgagcttaaa gtgctgaaac gcgcagaagg cgatggcgaa    1980
   ggcttcatcg ttattgatga cctggtggat accggtggta ctgcggttgc gattcgtgaa    2040
   atgtatccaa aagcgcactt tgtcaccatc ttcgcaaaac cggctggtcg tccgctggtt    2100
   gatgactatg ttgttgatat cccgcaagat acctggattg aacagccgtg ggatatgggc    2160
   gtcgtattcg tcccgccaat ctccggtcgc taatcttttc aacgcctggc actgccgggc    2220
   gttgttcttt ttaacttccc tgcataatta acgatgagtg tagatgctgt tattttgaac    2280
   cacagattag gattcttgat agagatgaga tgctcaatgg atcatcgtgt gatatgaaca    2340
   gacattgtat tatgatgaat ttacctgatg taggcgaatt tggatctagt atgttgggga    2400
   aatatgaacc tgacatgatt aagattgctc tttcggtggc tggtgagctc ggatctttta    2460
   ttctatactt aaaaaatgaa aataaataca aaggttcttg agggttgtgt taaattgaaa    2520
   gcgagaaata atcataaatt atttcattat cgcgatatcc gttaagtttg ctgcagctgg    2580
   atccatgggc gccagggcca gcgtgctgag cggaggcgag ctggacaggt gggagaagat    2640
   caggctgagg cctggaggca agaagaagta taagctgaag cacatcgtgt gggccagcag    2700
   ggagctggag aggttcgccg tgaaccctgg cctgctggag accagcgagg gctgcaggca    2760
   gatcctgggc cagctgcagc ccagcctgca gaccggcagc gaggagctga ggagcctgta    2820
   caacaccgtg gccaccctgt actgcgtgca ccagaggatc gagatcaagg acaccaagga    2880
   ggccctggac aagatcgagg aggagcagaa caagtccaag aagaaggccc agcaggctgc    2940
   tgccgacacc ggccacagca gccaggtgag ccagaactac cctatcgtgc agaacatcca    3000
   gggccagatg gtgcaccagg ccatcagccc taggaccctg aacgcctggg tgaaggtggt    3060
   ggaggagaag gccttcagcc ctgaggtgat ccctatgttc agcgccctga gcgagggagc 3120
   cacacctcag gacctgaaca ccatgctgaa caccgtggga ggccaccagg ccgccatgca 3180
   gatgctgaag gagaccatca acgaggaggc tgccgagtgg gacagggtgc accctgtgca 3240
   cgctggaccc atcgctccag gccagatgag ggagcccaga ggcagcgaca tcgccggcac 3300
   caccagcacc ctgcaggagc agatcggctg gatgaccaac aaccctccca tccctgtggg 3360
   cgaaatctac aagaggtgga tcatcctggg cctgaacaag atcgtgagga tgtacagccc 3420
   taccagcatc ctggatatca ggcagggccc taaagagccc ttcagggact acgtggacag 3480
   gttctacaag accctgagag ccgagcaggc cagccaggag gtgaagaact ggatgaccga 3540
   gaccctgctg gtgcagaacg ccaaccctga ctgcaagacc atcctgaagg ccctgggacc 3600
   tgctgccacc ctggaggaga tgatgaccgc ctgccagggc gtgggaggcc caggccacaa 3660
   ggccagggtg ctggccgagg ccatgagcca ggtgaccaac accgccacca tcatgatgca 3720
   gagaggcaac ttcaggaacc agaggaagat ggtgaagtgc ttcaactgcg gcaaggaggg 3780
   ccacaccgcc aggaactgca gggctcccag gaagaagggc tgctggaagt gcggcaagga 3840
   gggccaccag atgaaggact gcaccgagag gcaggccaac ttcctgggca agatctggcc 3900
   cagctacaag ggcaggccag gcaacttcct gcagagcagg cccgagccca ccgctccacc 3960
   tttcctgcag agcaggcccg agcccaccgc tcctcctgag gagagcttca ggagcggcgt 4020
   ggagacaacc acccctcctc agaagcagga gcccatcgac aaggagctgt accctctgac 4080
   cagcctgagg agcctgttcg gcaacgaccc tagcagccag gagtcgaccg ggccactaac 4140
   agaagaagca gagctagaac tggcagaaaa cagagagatt ctaaaagaac cagtacatgg 4200
   agtgtattat gacccatcaa aagacttaat agcagaaata cagaagcagg ggcaaggcca 4260
   atggacatat caaatttatc aagagccatt taaaaatctg aaaacaggaa tggagtggag 4320
   atttgattct agattagcat ttcatcacgt agctagagaa ttacatcctg aatattttaa 4380
   aaattgtaag cttatggcaa tattccaaag tagcatgaca aaaatcttag agccttttag 4440
   aaaacaaaat ccagacatag ttatctatca atacatggat gatttgtatg taggatctga 4500
   cttagaaata gggcagcata gaacaaaaat agaggagctg agacaacatc tgttgaggtg 4560
   gggacttaca accatggtag gttttccagt aacacctcaa gtacctttaa gaccaatgac 4620
   ttacaaagca gctgtagatc tttctcactt tttaaaagaa aaaggaggtt tagaagggct 4680
   aattcattct caacgaagac aagatattct tgatttgtgg atttatcata cacaaggata 4740
   ttttcctgat tggcagaatt acacaccagg accaggagtc agatacccat taacctttgg 4800
   ttggtgctac aagctagtac caatgattga gactgtacca gtaaaattaa agccaggaat 4860
   ggatggccca aaagttaaac aatggccatt gacagaagaa aaaataaaag cattagtaga 4920
   aatttgtaca gagatggaaa aggaagggaa aatttcaaaa attgggcctt aagcggccgc 4980
   cccgggagat ctcgatccgg aaagttttat aggtagttga tagaacaaaa tacataattt 5040
   tgtaaaaata aatcactttt tatactaata tgacacgatt accaatactt ttgttactaa 5100
   tatcattagt atacgctaca ccttttcctc agacatctaa aaaaataggt gatgatgcaa 5160
   ctttatcatg taatcgaaat aatacaaatg actacgttgt tatgagtgct tggtataagg 5220
   agcccaattc cattattctt ttagctgcta aaagcgacgt cttgtatttt gataattata 5280
   ccaaggataa aatatcttac gactctccat acgatgatct agttacaact atcacaatta 5340
   aatcattgac tgctagagat gccggtactt atgtatgtgc attctttatg acatcgccta 5400
   caaatgacac tgataaagta gattatgaag aatactccac agagttgatt gtaaatacag 5460
   atagtgaatc gactatagac ataatactat ctggatctac acattcaccg gaaactagtt 5520
   ctgagaaacc tgattatata gataattcta attgctcgtc ggtattcgaa atcgcgactc 5580
   cggaaccaat tactgataat gtagaagatc atacagacac cgtcacatac actagtgata 5640
   gcattaatac agtaagtgca tcatctggag aatccacaac agacgagact ccggaaccaa 5700
   ttactgataa agaagaagat catacagtta cagacactgt ctcatacact acagtaagta 5760
   catcatctgg aattgtcact actaaatcaa ccaccgatga tgcggatctt tatgatacgt 5820
   acaatgataa tgatacagta ccatcaacta ctgtaggcgg tagtacaacc tctattagca 5880
   attataaaac caaggacttt gtagaaatat ttggtattac cgcattaatt atattgtcgg 5940
   ccgtggcaat attctgtatt acatattata tatataataa acgttcacgt aaatacaaag 6000
<210> 5
   <211> 20
   <212> DNA
   <213> Artificial
<220>
   <223> primer
<400> 5
   catgacgagc ttccgagttc 20
<210> 6
   <211> 27
   <212> DNA
   <213> Artificial
<220>
   <223> primer
<400> 6
   gttgaagcac ttcaccatct tcctctg 27
<210> 7
   <211> 22
   <212> DNA
   <213> Artificial
<220>
   <223> primer
<400> 7
   cctgaacaag atcgtgagga tg 22
<210> 8
   <211> 20
   <212> DNA
   <213> Artifical
<400> 8
   gctccttata ccaagcactc 20